# EUROPÄISCHE PATENTSCHRIFT

(11) **EP 0 262 536 B1**
(45) Veröffentlichungstag und Bekanntmachung des Hinweises auf die Patenterteilung: **16.06.1993**
(21) Anmeldenummer: 87113739.4
(22) Anmeldetag: 19.09.1987
(51) Int. Cl.: A61N 5/06

(54) **Liegeplatte für eine Bestrahlungsliege**
Bed panel for radiation treatment bed
Plaque de couchette pour une couchette de radiothérapie

(30) Priorität: 25.09.1986 DE 3632527
(43) Veröffentlichungstag der Anmeldung: 06.04.1988
(73) Patentinhaber: Wolff, Friedrich, CH-4125 Riehen/Basel (CH)
(72) Erfinder: Wolff, Friedrich, CH-4125 Riehen/Basel (CH)
(74) Vertreter: Knoblauch, Ulrich, Dr.-Ing.

(56) Entgegenhaltungen:
- WO-A-86/03682
- DE-A- 2 817 908
- DE-A- 2 910 468
- DE-A- 2 911 758
- DE-A- 3 331 051
- DE-A- 3 544 833
- DE-U- 7 735 511

## Beschreibung

Die Erfindung bezieht sich auf eine Liegeplatte für eine Bestrahlungsliege mit einem Gehäuse, das Strahlungserzeuger, eine nach oben weisende Strahlenaustrittsöffnung und an deren Rändern sowie vorzugsweise auch zwischen den Rändern Stützelemente für die Liegeplatte aufweist, wobei die Liegeplatte für die Nutzstrahlung durchlässig ist, eine von einer Ebene abweichende Form hat und zum Abdecken der Strahlenaustrittsöffnung bestimmt ist.

Es sind Bestrahlungsliegen dieser Art bekannt (DE-OS 32 31 317), die zu Bräunungszwecken oder zur medizinischen Behandlung verwendet werden. Sie weisen mehrere nebeneinander angeordnete, stabförmige Leuchtstofflampen, die aufgrund des gewählten Leuchtstoffes und der Filtereigenschaften der Glashülle überwiegend UVA-Strahlung abgeben, und rinnenförmige Reflektoren auf. Die Liegeplatte ist der Form der Strahlenaustrittsöffnung angepaßt. Sie besteht aus einer ebenen Scheibe, wenn die Strahlenaustrittsöffnung in einer horizontalen Ebene liegt, oder aus einer muldenförmigen Scheibe mit über die Länge konstantem Querschnitt, wenn die Strahlenaustrittsöffnung nach beiden Seiten hin ansteigt. Die Liegeplatte ist zumindest an ihren beiden zueinander parallelen Seitenrändern sowie auf den Vorderkanten der Reflektoren abgestützt.

Die Liegeplatte besteht notwendigerweise aus hartem Material, beispielsweise Acrylglas. Von manchen Benutzern wird es als unangenehm empfunden, während der gesamten Bestrahlungsdauer auf einer harten, geraden Platte liegen zu müssen. Daher hat man bereits einen Polsterbelag auf der Liegeplatte vorgesehen (DE-PS 26 01 939), der aus für UVA-Strahlung durchlässigem Kunststoff mit luftgefüllten Taschen besteht.

Die vorliegende Erfindung hat das Ziel, dem Benutzer auch ohne Polsterauflage ein bequemeres Liegen zu ermöglichen. Die Aufgabe besteht darin, eine Liegeplatte der eingangs beschriebenen Art mit einer neuartigen, ein entspanntes Liegen ermöglichenden Form anzugeben.

Diese Aufgabe wird erfindungsgemäß gelöst durch einen ersten Abschnitt, der sich im Bereich der Längsmitte befindet und die tiefste Stelle der Liegefläche bildet, hieran in Längsrichtung anschließende zweite und dritte Abschnitte, die zum Kopfende bzw. zum Fußende hin ansteigen, und randseitige Abschnitte mit zueinander parallelen Seitenrändern, die sich zu beiden Seiten der ersten bis dritten Abschnitte erstrecken und über Höhenunterschiede ausgleichende Zwischenwände mit ihnen verbunden sind.

Bei dieser Konstruktion befindet sich die Körpermitte eines auf dem Rücken liegenden Benutzers an der tiefsten Stelle der Liegeplatte. Rücken und Kopf befinden sich auf dem allmählich ansteigenden zweiten Abschnitt, die Beine auf dem allmählich ansteigenden dritten Abschnitt. Bei dieser Verteilung hat der Körper eine natürliche, entspannte Lage. Die einzelnen Körperpartien sind gleichmäßig belastet. Der Benutzer findet es auf einer solchen Liegeplatte auch nach 15 oder 30 Minuten noch sehr angenehm.

Die randseitigen Abschnitte können über eine größere Länge auf randseitigen Stützelementen des Gehäuses abgestützt sein. Über die Zwischenwände werden dann die übrigen Abschnitte gut gehalten. Sie benötigen daher keine oder wenig zusätzliche Unterstützung.

Günstig ist ein vierter Abschnitt, der sich zwischen dem dritten Abschnitt und dem Fußende befindet und eine in Längsrichtung etwa gleichbleibende Höhe hat. Zwischen dem dritten Abschnitt, der die Oberschenkel aufnimmt, und dem vierten Abschnitt, der die Unterschenkel aufnimmt, ergibt sich eine Neigungsänderung, die der natürlichen Beinhaltung entspricht.

Das Gehäuse einer üblichen Bestrahlungsliege mit stabförmigen Leuchtstofflampen hat eine Strahlenaustrittsöffnung, deren durch die Ränder bestimmte Querschnittsform in Längsrichtung konstant ist. Die Stützelemente, seien sie am Rand oder zwischen den Lampen angeordnet, enden in Höhe dieser Strahlenaustrittsöffnung. Es ist ein erstrebenswertes Ziel, die neue Liegeplatte so auszugestalten, daß sie in Verbindung mit diesen bekannten Gehäusen verwendet werden kann.

Zu diesem Zweck können an Kopf- und/oder Fußende abgebogene Stirnwände vorgesehen sein, die bis in die Höhe des ersten Abschnitts ragen. Eine solche Liegeplatte ist zumindest am Kopfende und am Fußende und im Bereich des ersten Abschnitts auf den üblichen Stützelementen gelagert.

Es empfiehlt sich, daß an der Unterseite zumindest eines der zweiten bis vierten Abschnitte eine Stützanordnung mit unterschiedlicher Stützhöhe vorgesehen ist. Damit wird der Höhenänderung der einzelnen Abschnitte Rechnung getragen.

Insbesondere kann die Stützanordnung mindestens einen längs verlaufenden Steg aufweisen, der zwischen den als stabförmige Lampen ausgebildeten Strahlungserzeugern angeordnet ist.

Dem gleichen Zweck dient es, wenn an der Unterseite zumindest eines der zweiten bis vierten Abschnitte ein quer laufender Stützsteg angebracht ist, der bis in die Höhe des ersten Abschnitts ragt.

Für eine Bestrahlungsliege, deren Strahlenaustrittsöffnung nach beiden Seiten hin ansteigt und über die Länge konstante Querschnittsform hat, empfiehlt es sich, daß die randseitigen Abschnitte, ausgehend vom ersten Abschnitt, anschließen, die zum Seitenrand hin ansteigen. Die beiden letztgenannten Abschnitte dienen der Anpassung an die Form der Strahlenaustrittsöffnung und können unmittelbar auf üblichen Stützelementen ruhen.

Unter Ausnutzung dieser Zwischenwände kann zumindest einer der zweiten, dritten und vierten Abschnitte in der Höhe über den Seitenrand der randseitigen Abschnitte hinausragen. Dies erlaubt es, eine gewünschte Neigung der zweiten und dritten Abschnitte unabhängig von der Form des vorhandenen üblichen Gehäuses vorzusehen und hierbei verhältnismäßig große Neigungen zu vermeiden.

Bei einer bevorzugten Ausführungsform ist dafür gesorgt, daß die ersten bis vierten und die randseitigen Abschnitte im wesentlichen eben sind. Dies erleichtert die Herstellung und ermöglicht auf einfache Weise eine vielfache Unterstützung der Liegeplatte.

Ferner kann im zweiten Abschnitt eine Vertiefung zur Plazierung eines Kopfkissens vorgesehen sein. Da die Liegeplatte ohnehin geformt werden muß, kann auch eine solche Vertiefung ohne zusätzlichen Aufwand mit eingeformt werden.

Es empfiehlt sich, daß das Kopfkissen eine größere Breite als die Vertiefung und seitlich angehobenene Randbereiche hat. Wegen dieser Randbereiche hat das Kopfkissen eine sichere Lage. Außerdem ergibt sich in der Mitte eine längsgerichtete Mulde, welche eine sehr bequeme Auflage für den Kopf bildet.

Besonders günstig ist die Anwendung auf eine mit einem Deckengerät kombinierte Bestrahlungsliege. Die Gesamtbestrahlung kann dann in der bequemen Rückenlage erfolgen.

Die Erfindung wird nachstehend anhand eines in der Zeichnung dargestellten, bevorzugten Ausführungsbeispiels näher erläutert. Es zeigen:
- Fig. 1: einen Querschnitt längs der Linie A-A der Fig. 3 durch eine Bestrahlungsliege gemäß der Erfindung,
- Fig. 2: einen Längsschnitt durch die verwendete Liegeplatte in verkleinertem Maßstab,
- Fig. 3: eine Draufsicht auf die Liegeplatte in verkleinertem Maßstab.
- Fig. 4: in vergrößerter Darstellung das Kopfende der Liegeplatte,
- Fig. 5: einen Querschnitt durch ein Kopfkissen und
- Fig. 6: in schematischer Darstellung eine Kombination einer erfindungsgemäß ausgestalteten Bestrahlungsliege mit einem Deckengerät.

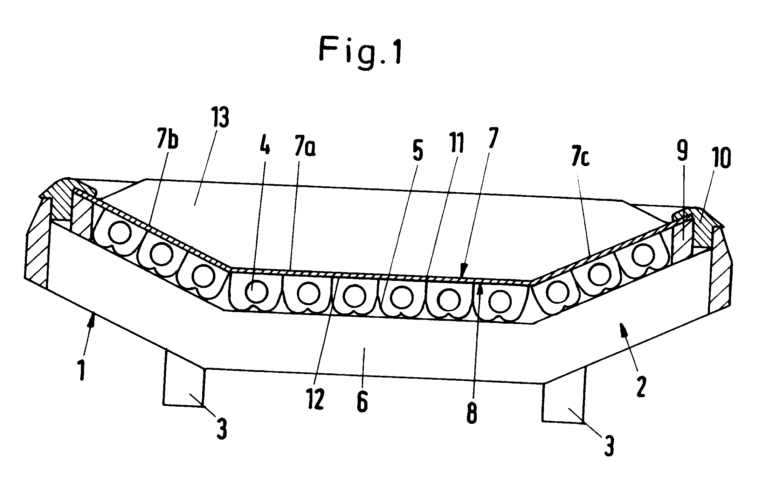

Fig. 1 zeigt eine Bestrahlungsliege 1 mit einem Gehäuse 2, das auf Füßen 3 steht. Es weist als Strahlungserzeuger zehn parallel nebeneinander angeordnete stabförmige Leuchtstofflampen 4 auf, die je von einem rinnenförmigen Reflektor 5 umgeben sind. Die Reflektoren sind auf mehreren in Längsrichtung gegeneinander versetzten Trägern 6 gehalten. Die Stirn- und Seitenränder des Gehäuses 2 definieren an ihrer Oberseite eine Strahlenaustrittsöffnung 7, deren Querschnittsform über die Längsrichtung der Bestrahlungsliege 1 konstant ist und einen horizontalen Abschnitt 7a sowie zwei zur Seite hin ansteigende Abschnitte 7b und 7c aufweist. Die Lampen 4 sind dicht unter dieser Strahlenaustrittsöffnung angeordnet. Die Reflektoren 5 reichen bis an die Abschnitte der Strahlenaustrittsöffnung.

Diese Strahlenaustrittsöffnung 7 wird durch eine Liegeplatte 8 abgedeckt, die in der veranschaulichten Querschnittslage der Form dieser Strahlenaustrittsöffnung 7 genau angepaßt ist. Die Liegeplatte 8 ist durch randseitige Stützelemente 9, gegen die sie mit Klemmelementen 10 gehalten ist, und durch dazwischen angeordnete Stützelemente 11, welche durch die Vorderkanten der Reflektoren 5 gebildet sind, abgestützt.

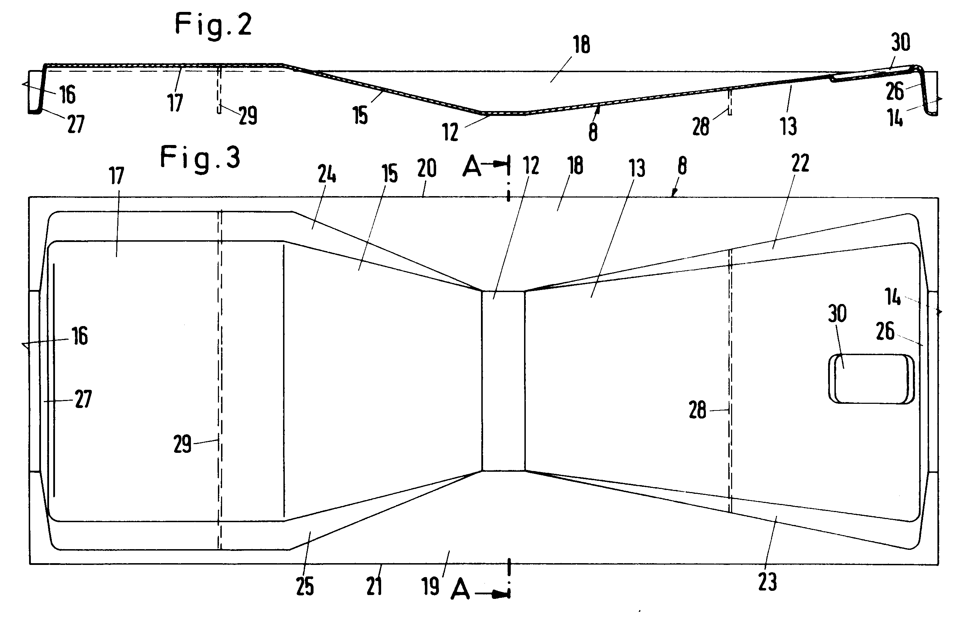

Die Fig. 2 und 3 zeigen die Liegeplatte 8 im Längsschnitt und in Draufsicht. Im Bereich der Längsmitte gibt es einen horizontalen ersten Abschnitt 12, der rechteckförmig ausgebildet ist. Auf der einen Seite schließt ein ebenfalls ebener zweiter Abschnitt 13 an, der zum Kopfende 14 hin ansteigt. Auf der anderen Seite schließt ein ebener dritter Abschnitt 15 an, der zum Fußende 16 hin ansteigt. Ein vierter ebener Abschnitt 17 erstreckt sich horizontal zwischen dem dritten Abschnitt 15 und dem Fußende 16. Zu beiden Seiten des ersten Abschnitts 12 befinden sich zwei randseitige Abschnitte, nämlich ein fünfter Abschnitt 18 und ein sechster Abschnitt 19, die der Querschnittsform der Strahlenaustrittsöffnung im Bereich der Abschnitte 7b und 7c folgen, zum Seitenrand 20 bzw. 21 hin also ansteigen, und außerdem den zweiten Abschnitt 13 und den dritten Abschnitt 15 seitlich überlappen. Zwischen dem zweiten Abschnitt 13 und dem fünften Abschnitt 18 erstreckt sich eine Zwischenwand 22, zwischen dem zweiten Abschnitt 13 und dem sechsten Abschnitt 19 eine Zwischenwand 23, zwischen dem dritten Abschnitt 15 und dem fünften Abschnitt 18 sowie zwischen dem vierten Abschnitt 17 und dem Seitenrand 20 eine Zwischenwand 24 und zwischen dem dritten Abschnitt 15 und dem sechsten Abschnitt 19 sowie dem vierten Abschnitt 17 und dem Seitenrand 21 eine Zwischenwand 25, mit der die jeweiligen Höhenunterschiede ausgeglichen werden.

An der Kopfseite 14 gibt es eine Stirnwand 26, die nach unten abgebogen ist und bis in die Höhe des ersten Abschnitts 12 reicht. Eine entsprechende Stirnwand 27 ist am Fußende 16 vorgesehen. Infolgedessen hat die Liegeplatte ringsum an ihrem Rand eine Auflage auf Stützelementen 9 des Gehäuses. Sie wird überdies im ersten Abschnitt 12 und im fünften und sechsten Abschnitt 18, 19 von den Stützelementen 11, also den Reflektorkanten, abgestützt. Falls dies noch nicht genügen sollte, können die gestrichelt angedeuteten quer laufenden Stützstege 28 und 29 am zweiten und vierten Abschnitt angebracht werden. Sie können aus dem gleichen Material wie die Liegeplatte 8, also beispielsweise aus Acrylglas, bestehen und werden vorzugsweise angeklebt. Die Stützstege 28, 29 bilden demnach eine Stützanordnung mit unterschiedlicher Stützhöhe.

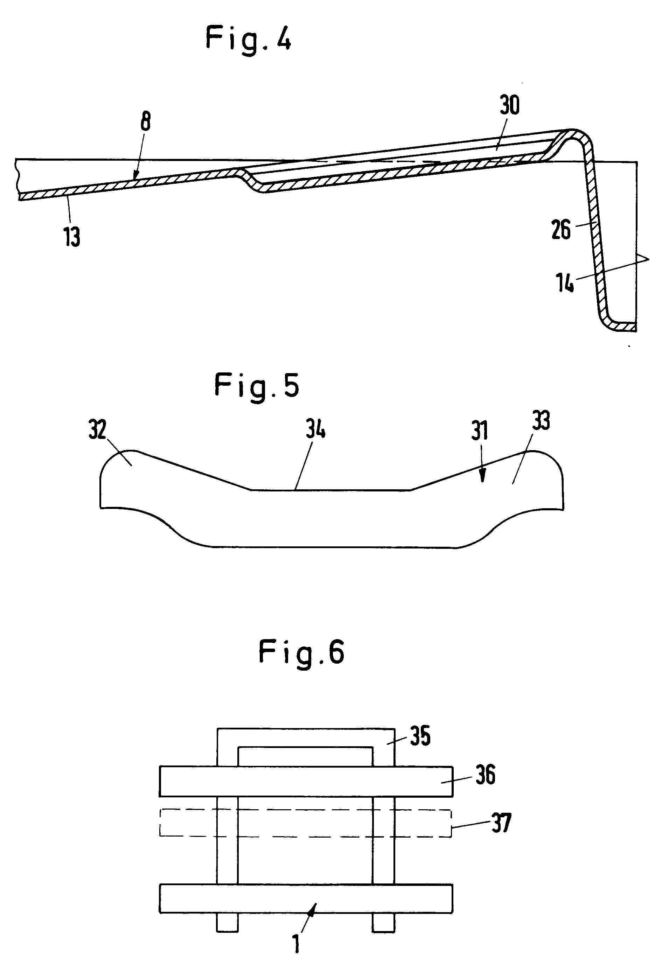

Wie in Fig. 4 noch deutlicher gezeigt ist, gibt es im Kopfbereich des zweiten Abschnitts 13 eine Vertiefung 30, die zum Einlegen eines Kopfkissens 31 dient. Dieses Kopfkissen besteht beispielsweise aus Polyurethanschaum. Es ist breiter als die Vertiefung 30 und liegt daher mit seinen angehobenen Randbereichen 32 und 33 neben der Vertiefung auf der Liegeplatte 8 auf. Dies ergibt einen sicheren Halt. Außerdem entsteht auf diese Weise eine Längsmulde 34, die den Kopf des Benutzers gut abzustützen vermag, Fig. 5.

Fig. 6 zeigt eine Kombination, in der an einem gemeinsamen Gestell 35 sowohl eine Bestrahlungsliege 1 gemäß der Erfindung als auch ein Bestrahlungs-Deckengerät 36 montiert sind. Das Deckengerät hat eine ähnliche Anordnung der Leuchtstofflampen 4 und der Reflektoren 5, strahlt aber nach unten. Wenn sich ein Benutzer auf die Bestrahlungsliege 1 gelegt hat, kann das Deckengerät 36 motorisch aus der vollausgezogenen Stellung in die gestrichelt gezeichnete Stellung 37 abgesenkt werden, so daß die auf der Bestrahlungsliege 1 plazierte Person gleichzeitig von oben und unten bestrahlt werden kann, während sie eine bequeme Liegehaltung einnimmt.

Von der veranschaulichten Konstruktion kann in vielfacher Hinsicht abgewichen werden, ohne den Grundgedanken der Erfindung zu verlassen. Beispielsweise kann die Liegeplatte 8 zwischen den Rändern auch auf anderen Stützelementen als den Stützelementen 11 abgestützt werden. Beispielsweise kann zwei oder drei Leuchtstofflampen 4 je ein gemeinsamer Reflektor zugeordnet werden, so daß die Abstützung in größeren Abständen erfolgt. Es ist auch möglich, anstelle der Reflektorseitenwände andere Stützelemente vorzusehen, beispielsweise zwischen den Lampen 4 angeordnete Acrylglasstreifen oder sogar nur einzelne Stützsäulen. Die Zahl der Lampen 4 kann beliebig variiert werden. Anstelle dieser Lampen können aber auch Hochdruckbrenner oder andere Strahlungserzeuger eingesetzt werden. Dies gilt auch für das Deckengerät 36. Anstelle der ebenen Abschnitte 12, 13, 15, 17, 18 und 19 können auch gerundete Abschnitte vorgesehen werden, sofern dies im Hinblick auf die Form der Strahlenaustrittsöffnung bzw. der durch die Stützelemente vorgegebenen Fläche erforderlich ist. Der erste Abschnitt 12 ist im Ausführungsbeispiel wesentlich kürzer als die zweiten bis vierten Abschnitte. Im Extremfall bildet der erste Abschnitt nur einen schmalen Übergangsbereich zwischen zweitem und drittem Abschnitt. Wie das Ausführungsbeispiel zeigt, braucht der erste Abschnitt nicht genau in Längsmitte vorgesehen zu sein, sondern kann hierzu auch etwas versetzt sein.

## Patentansprüche

1. Liegeplatte (8) für eine Bestrahlungsliege (1) mit einem Gehäuse (2), das Strahlungserzeuger, eine nach oben weisende Strahlenaustrittsöffnung (7) und an deren Rändern sowie vorzugsweise auch zwischen den Rändern Stützelemente (9, 11) für die Liegeplatte aufweist, wobei die Liegeplatte (8) für die Nutzstrahlung durchlässig ist, eine von einer Ebene abweichende Form hat und zum Abdecken der Strahlenaustrittsöffnung (7) bestimmt ist, gekennzeichnet durch einen ersten Abschnitt (12), der sich im Bereich der Längsmitte befindet und die tiefste Stelle der Liegefläche bildet, hieran in Längsrichtung anschließende zweite und dritte Abschnitte (13, 15), die zum Kopfende (14) bzw. zum Fußende (16) hin ansteigen, und randseitige Abschnitte (18, 19) mit zueinander parallelen Seitenrändern (20, 21), die sich zu beiden Seiten der ersten bis dritten Abschnitte (12, 13, 15) erstrecken und über Höhenunterschiede ausgleichende Zwischenwände (22, 23, 24, 25) mit ihnen verbunden sind.

2. Liegeplatte nach Anspruch 1, gekennzeichnet durch einen vierten Abschnitt (17), der sich zwischen dem dritten Abschnitt (15) und dem Fußende (16) befindet und eine in Längsrichtung etwa gleichbleibende Höhe hat.

3. Liegeplatte nach Anspruch 1 oder 2, dadurch gekennzeichnet, daß an Kopf- und/oder Fußende (14, 16) abgebogene Stirnwände (26, 27) vorgesehen sind, die bis in die Höhe des ersten Abschnitts (12) ragen.

4. Liegeplatte nach einem der Ansprüche 1 bis 3, dadurch gekennzeichnet, daß an der Unterseite zumindest eines der zweiten bis vierten Abschnitte (13, 15, 17) eine Stützanordnung (28, 29) mit unterschiedlicher Stützhöhe vorgesehen ist.

5. Liegeplatte nach Anspruch 4, dadurch gekennzeichnet, daß die Stützanordnung mindestens einen längs verlaufenden Steg aufweist, der zwischen den als stabförmige Lampen ausgebildeten Strahlungserzeugern angeordnet ist.

6. Liegeplatte nach einem der Ansprüche 1 bis 4, dadurch gekennzeichnet, daß an der Unterseite zumindest eines der zweiten bis vierten Abschnitte (13, 15, 17) ein quer laufender Stützsteg (28, 29) angebracht ist, der bis in die Höhe des ersten Abschnitts (12) ragt.

7. Liegeplatte für eine Bestrahlungsliege, deren Strahlenaustrittsöffnung nach beiden Seiten hin ansteigt und über die Länge konstante Querschnittsform hat, nach einem der Ansprüche 1 bis 6, dadurch gekennzeichnet, daß die randseitigen Abschnitte (18, 19) ausgehend vom ersten Abschnitt (13) zum Seitenrand (20, 21) hin ansteigen.

8. Liegeplatte nach einem der Ansprüche 1 bis 7, dadurch gekennzeichnet, daß zumindest einer der zweiten, dritten und vierten Abschnitte (12, 13, 15) in der Höhe über den Seitenrand (20, 21) der randseitigen Abschnitte (18, 19) hinausragt.

9. Liegeplatte nach einem der Ansprüche 1 bis 8, dadurch gekennzeichnet, daß die ersten bis vierten und die randseitigen Abschnitte (12, 13, 15, 17, 18, 19) im wesentlichen eben sind.

10. Liegeplatte nach einem der Ansprüche 1 bis 9, dadurch gekennzeichnet, daß im zweiten Abschnitt (13) eine Vertiefung (30) zur Plazierung eines Kopfkissens (31) vorgesehen ist.

11. Liegeplatte nach Anspruch 10, dadurch gekennzeichnet, daß das Kopfkissen (31) eine größere Breite als die Vertiefung (30) und seitlich angehobene Randbereiche (32, 33) hat.

12. Liegeplatte nach einem der Ansprüche 1 bis 11, gekennzeichnet durch die Anwendung auf eine mit einem Deckengerät (36) kombinierte Bestrahlungsliege (1).

## Claims

1. Reclining top (8) for a sun bed (1) with a housing (2) which comprises radiation sources, an upwardly pointing beam outlet opening (7) and, at the edges thereof as well as preferably also between the edges, supporting elements (9, 11) for the reclining top, wherein the reclining top (8) is permeable to effective radiation, has a shape deviating from a plane and is designed to cover the beam outlet opening (7), characterised by a first section (12) which is located in the region of the longitudinal centre and forms the lowermost point of the reclining surface, second and third sections (13, 15) adjoining it in the longitudinal direction and sloping up towards the head end (14) and foot end (16) respectively, and edge sections (18, 19) with side edges (20, 21) parallel to each other, which extend on both sides of the first to third sections (12, 13, 15) and are connected thereto by partitions (22, 23, 24, 25) which level out differences in height.

2. Reclining top according to claim 1, characterised by a fourth section (17) which is located between the third section (15) and the foot end (16) and has a more or less constant height in the longitudinal direction.

3. Reclining top according to claim 1 or 2, characterised in that angled end walls (26, 27) which extend as far as the height of the first section (12) are provided at the head and/or foot end (14, 16).

4. Reclining top according to any of claims 1 to 3, characterised in that on the underside of at least one of the second to fourth sections (13, 15, 17) there is provided a supporting arrangement (28, 29) with different supporting heights.

5. Reclining top according to claim 4, characterised in that the supporting arrangement comprises at least one longitudinally extending web which is arranged between the radiation sources designed as rod-shaped lamps.

6. Reclining top according to any of claims 1 to 4, characterised in that on the underside of at least one of the second to fourth sections (13, 15, 17) is mounted a transversely extending supporting web (28, 29) which extends as far as the height of the first section (12).

7. Reclining top for a sun bed, of which the beam outlet opening slopes up towards both sides and has a constant cross-sectional shape over the length, according to any of claims 1 to 6, characterised in that the edge sections (18, 19), starting from the first section (13), slope up towards the side edge (20, 21).

8. Reclining top according to any of claims 1 to 7, characterised in that at least one of the second, third and fourth sections (12, 13, 15) protrudes in height beyond the side edge (20, 21) of the edge sections (18, 19).

9. Reclining top according to any of claims 1 to 8, characterised in that the first to fourth and the edge sections (12, 13, 15, 17, 18, 19) are essentially flat.

10. Reclining top according to any of claims 1 to 9, characterised in that in the second section (13) is provided a recess (30) for placing a pillow (31).

11. Reclining top according to claim 10, characterised in that the pillow (31) has a greater width than the recess (30) and laterally raised edge regions (32, 33).

12. Reclining top according to any of claims 1 to 11, characterised by application to a sun bed (1) combined with a ceiling appliance (36).

## Revendications

1. Plaque (8) pour une couchette de radiothérapie (1) comprenant une coque (2), le générateur de rayonnement, une ouverture de sortie de rayonnement (7) tournée vers le haut et des éléments d'appui (9, 11) pour la plaque de couchette sur ses bords ainsi que de préférence également entre les bords, la plaque de couchette (8) laissant passer le rayonnement utile, ayant une forme différente d'un plan et étant prévue pour recouvrir l'ouverture de sortie de rayonnement (7), caractérisée par une première section (12) prévue dans la région du centre longitudinal et qui forme la position la plus profonde de la surface de la couchette, une seconde et une troisième sections (13, 15) qui s'y raccordent en direction longitudinale, qui vont en montant en direction de l'extrémité destinée à la tête (14) ou de l'extrémité destinée aux pieds (16), et des sections de bord (18, 19) à bords latéraux parallèles (20, 21) qui s'étendent de chaque côté de la première à la troisième section (12, 13, 15) et qui sont reliées à ces dernières par des parois intermédiaires (22, 23, 24, 25) d'adaptation et de hauteurs différentes.

2. Plaque de couchette selon la revendication 1, caractérisée par une quatrième section (17) qui se trouve entre la troisième section (15) et l'extrémité destinée aux pieds (16) et présente en direction longitudinale une hauteur sensiblement invariable.

3. Plaque de couchette selon la revendication 1 ou 2, caractérisée en ce que des parois frontales coudées (26, 27) sont prévues aux extrémités destinées à la tête et/ou aux pieds (14, 16), qui font saillie jusqu'à la hauteur de la première section (12).

4. Plaque de couchette selon l'une quelconque des revendications 1 à 3, caractérisée en ce qu'un agencement d'appui (28, 29) à hauteurs d'appui variables est prévu sur le côté inférieur d'au moins l'une parmi les seconde à quatrième sections (13, 15, 17).

5. Plaque de couchette selon la revendication 4, caractérisée en ce que l'agencement d'appui comprend au moins une branche orientée longitudinalement et disposée entre les générateurs de rayonnement constitués par des lampes en forme de barres.

6. Plaque de couchette selon l'une quelconque des revendications 1 à 3, caractérisée en ce que sur le côté inférieur d'au moins l'une parmi les seconde à quatrième sections (13, 15, 17) est montée une branche d'appui (28, 29) orientée transversalement qui parvient jusqu'à la hauteur de la première section.

7. Plaque de couchette pour couchette de radiothérapie dont l'ouverture de sortie de rayonnement va en montant de chaque côté et présente une forme en section constante sur sa longueur, selon l'une quelconque des revendications 1 à 6, caractérisée en ce que les sections latérales de bord (18, 19) vont en montant en partant de la première section (13) vers les bords latéraux (20, 21).

8. Plaque de couchette selon l'une quelconque des revendications 1 à 7, caractérisée en ce que l'une au moins parmi les seconde, troisième et quatrième sections (12, 13, 15) dépasse en hauteur le bord latéral (20, 21) des sections latérales de bord (18, 19).

9. Plaque de couchette selon l'une quelconque des revendications 1 à 8, caractérisée en ce que la première à la quatrième section ainsi que les sections de bord (12, 13, 15, 16, 18, 19) sont essentiellement planes.

10. Plaque de couchette selon l'une quelconque des revendications 1 à 9, caractérisée en ce qu'il est prévu dans la seconde section (13) une partie creuse (30) permettant d'y placer un oreiller (31).

11. Plaque de couchette selon la revendication 10, caractérisée en ce que l'oreiller (13) est plus large que la partie creuse (30) et en ce que ses régions de bord latérales (32, 33) sont surélevées.

12. Plaque de couchette selon l'une quelconque des revendications 1 à 11, caractérisée par l'utilisation d'une couchette de radiothérapie (1) combinée à un appareil en surplomb (36).
